# EUROPEAN PATENT APPLICATION

(11) **EP 4 806 874 A1**
(43) Date of publication of application: **16.09.2026**
(21) Application number: 25162888.9
(22) Date of filing: 11.03.2025
(51) Int. Cl.: C07B 31/00, C07C 1/213, B01J 32/00

(54) **IRON-CATALYSED HYDRODEOXYGENATION ENABLED BY MAGNETOCATALYSIS**

(71) Applicant: Max-Planck-Gesellschaft zur Förderung der Wissenschaften e.V., 80539 München (DE)
(72) Inventor: Ahmedi, Sihana, 45470 Mülheim an der Ruhr (DE); Bordet, Alexis, 45470 Mülheim an der Ruhr (DE); Leitner, Walter, 45470 Mülheim an der Ruhr (DE)
(74) Representative: Hoffmann Eitle

(57) **Abstract**

The present invention is directed towards catalysts comprising iron-containing particles, as well as methods of completing hydrodeoxygenation reactions utilising such catalysts. In particular, a method employing such catalysts to complete the hydrodeoxygenation of esters is claimed. The catalysts and methods of the present invention improve upon the disadvantages associated with literature hydrodeoxygenation reactions and in particular, allow hydrodeoxygenation reactions under mild conditions with good substrate scope, high yields and high selectivity.

## Description

### Field of the Invention

The present invention is directed towards methods of iron-catalysed hydrodeoxygenation reactions, particularly hydrodeoxygenation reactions of esters. The present invention is further directed towards the use of iron-containing catalysts in hydrodeoxygenation reactions, particularly hydrodeoxygenation reactions of esters.

### Background of the Invention

The reduction of esters to methyl-containing products is a key transformation enabling the preparation of building blocks in synthetic chemistry as well as the valorization of waste oil and polyesters (e.g. PET). It is typically carried out using stoichiometric or excess amounts of powerful reducing agents such as lithium aluminum hydride (LiAlH₄) and sodium borohydride (NaBH₄) under harsh conditions. Poor atom economy, limited functional group tolerance and safety concerns are severe limitations, however. Catalytic approaches are much scarcer than for transformations targeting the reduction of esters to aldehydes, ethers, or alcohols. Recent developments involved catalytic approaches relying mainly on hydroboration and hydrosilylation using metal complexes. For example, Cook et al. (Cook, A., Prakash, S., Zheng, Y.-L. & Newman, S. G. Exhaustive reduction of esters enabled by nickel catalysis. Journal of the American Chemical Society 142, 8109-8115 (2020).) reported on the reduction of aryl esters to toluene derivatives in the presence of 10 mol% of molecular Ni(cod)₂ via hydrosylilation through the presence of various additives (e.g. 20 mol% of Icy.HBF4, 1 equivalent of KOtBu at 110 °C for 10 h with 36-82 % of yield ranges obtained). Han et al. (Han, B., Ren, C., Jiang, M. & Wu, L. Titanium-Catalyzed Exhaustive Reduction of Oxo-Chemicals. Angewandte Chemie International Edition 61, e202209232 (2022).) reported the hydroboration of methylbenzoate with ammonia borane using a molecular Ti-based catalyst (Cp₂TiCl₂, 10 mol%) in the presence of MeOLi (1 equivalent) at 120 °C for 36 h. Sahu et al. (Sahu, M. K., Pattanaik, S. & Gunanathan, C. Cobalt-catalyzed reduction of esters to alkanes. Chemical Communications 61, 1661-1164(2025).) reported the hydrosylilation of methylbenzoate using a molecular Co-based catalyst ((NNNHtBu)CoBr₂, 5 mol%) in the presence of KOtBu (40 mol%) at 100 °C for 12 h. Approaches using directly H₂ as a reducing agent have been reported in heterogeneous catalysis, where research focused so far mainly on the reduction of long chain aliphatic esters using Pt-based catalysts under harsh conditions.

Traditional methods of hydrodeoxygenation reactions are coupled with significant drawbacks, including the need for expensive reagents, toxic reagents, low selectivity, poor chemoselectivity, need for multistep synthetic routes, poor atom economy/efficiency (e.g., stoichiometric/excess amounts of reducing agents such as LiAlH₄, or hydroboration/ hydrosilylation reactions generating large amounts of wastes), narrow range of functional group tolerance (e.g., when using LiAlH₄ or related reagents, as well as most heterogeneous catalysts that were so far mainly developed for long chain aliphatic esters (waste oil treatment)), and catalysts not being recyclable.

To avoid the disadvantages associated with past methods, the present inventors have developed a method of hydrodeoxygenation of substrates using magnetically induced catalysis and H₂ as a reducing agent, without the use of any additive.

Magnetic induction as an alternative to conventional heating provides great potential for energy efficiency and suitability for intermittence by the usage of catalysts designed to combine high magnetic heating efficiency (SAR), and good catalytic activity via heat originating directly from magnetic nanoparticles by an extremely localized, rapid, energy-efficient manner.

It is an object of the present invention to provide a method with which a substrate containing an alcohol, an aldehyde, a ketone and/or an ester functionality (particularly an ester) can undergo a hydrodeoxygenation reaction to the corresponding alkane. It is a further object of the present invention, to improve upon the disadvantages associated with conventional methods. In particular, the present invention allows for the production of alkanes from esters (and/or alcohols and/or aldehydes and/or ketones) with good yields, good selectivity and good substrate scope, whilst the catalyst according to the present invention is stable and recyclable, and H₂ is used as a reducing agent, without the use of any additive. Particularly, the present invention allows for hydrodeoxygenation of compounds containing an aromatic ring, wherein the aromatic ring is conserved. Furthermore, the hydrodeoxygenation reaction according to the present invention proceeds under mild reaction conditions (low pressures and mild temperatures, including ambient pressures), is efficient, and is sustainable. The herein described method also provides for a potential adaptivity of the catalytic process to intermittent power supply.

In addition, the catalysts described herein are stable and recyclable. They allow for rapid, localised and energy efficient heating due to magnetically induced catalysis. This contributes to the potential adaptivity of the catalytic process to intermittent power supply, to sustainability, to mild reaction conditions (*e*.*g*., <5 bar H₂), and to efficiency of the reactions.

Additionally, the catalysts described herein allow for the hydrodeoxygenation of substrates, without using expensive and/or rare metals, such as the noble metals. The catalysts described herein also allow for hydrodeoxygenation reactions which are high yielding, and very selective.

The presently claimed invention also allows for the hydrodeoxygenation of substrates, in particular alcohol, aldehyde, ketone and/or ester substrates (preferably ester substrates), with H₂ pressures below 5 bar (mild conditions). This is also greatly beneficial from a practical and safety point of view and avoids the need for specialised highpressure technologies on laboratory or production scale.

The presently claimed invention also allows for hydrodeoxygenation reactions which are high-yielding, and highly selective. The invention allows for the complete reduction of ester substrates to the corresponding alkanes in high yields and with high selectivity.

The present invention also relates to iron-containing nanoparticles, for example iron carbide nanoparticles, which are used for the exhaustive reduction of esters (ester to the corresponding alkane) and which exhibit high activity, selectivity, and stability for the reduction of a large scope of esters under mild conditions (3 bar H₂) as well as showing adaptability to the intermittent power supply.

### Summary of the Invention

The above object is solved by a method of hydrodeoxygenation of a substrate, the method comprising bringing a substrate (e.g., an ester) and hydrogen into contact with a catalyst and applying an alternating current magnetic field, as defined in claim 1 and claim 2. It is also solved by the use of a catalyst comprising iron-containing nanoparticles in hydrodeoxygenation reactions, as defined in claim 14. Preferred embodiments are the subject of the dependent claims.

By employing the methods and/or catalyst according to the present invention, the inventors have improved upon the drawbacks associated with traditional methods of hydrodeoxygenation reactions, particularly ester hydrodeoxygenation reactions.

### Brief Description of the Drawings

**Figure 1****:** Synthesis and characterization of iron carbide nanoparticles. a) Synthetic approach, b) STEM-HAADF and TEM images, c) PXRD, d) Mössbauer Analysis Test e) VSM measurement and f) XPS of the catalyst.
**Figure 2****:** PXRD analysis of starting Fe(0) nanoparticles.

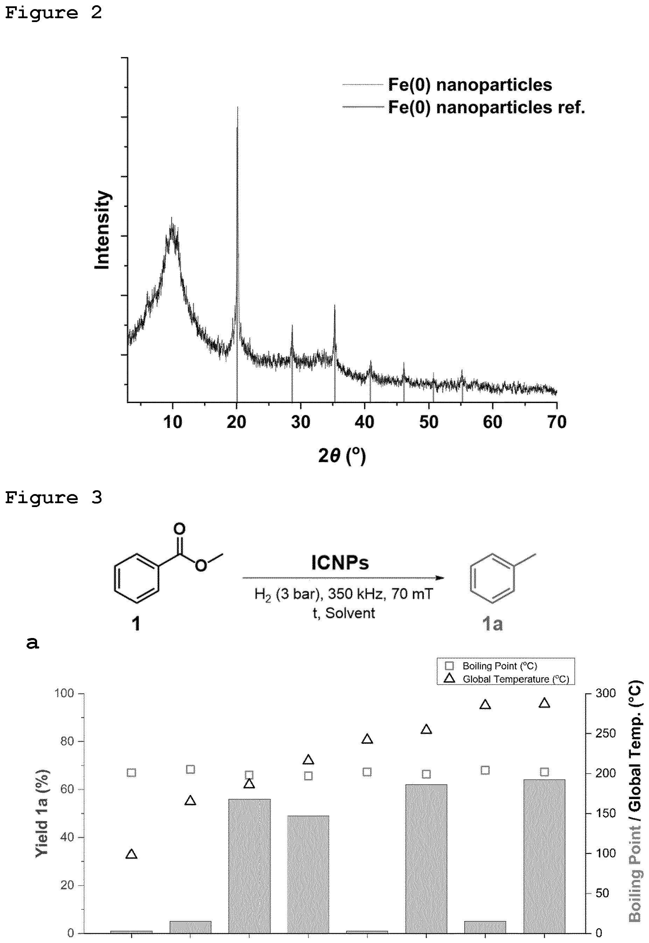

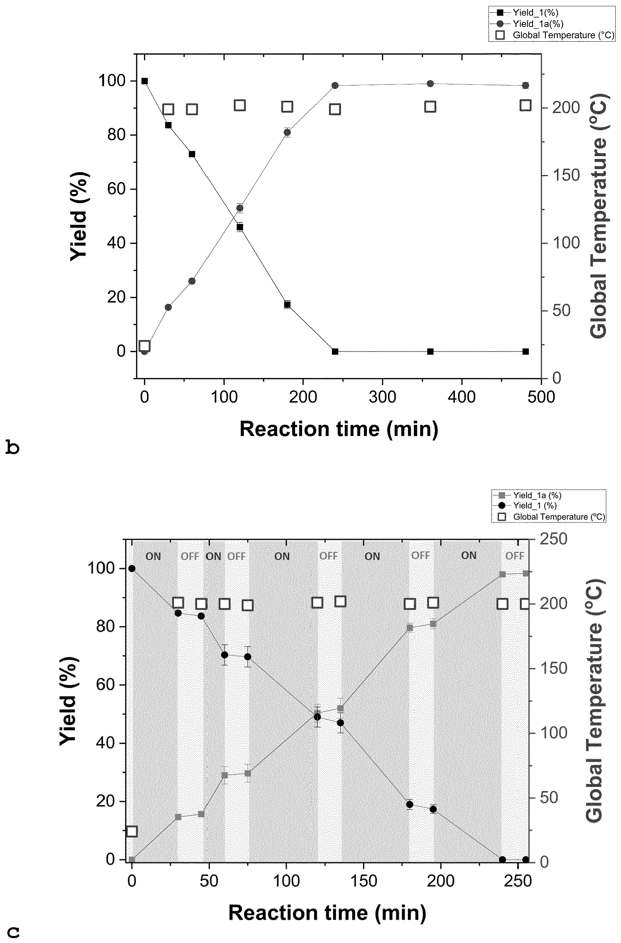

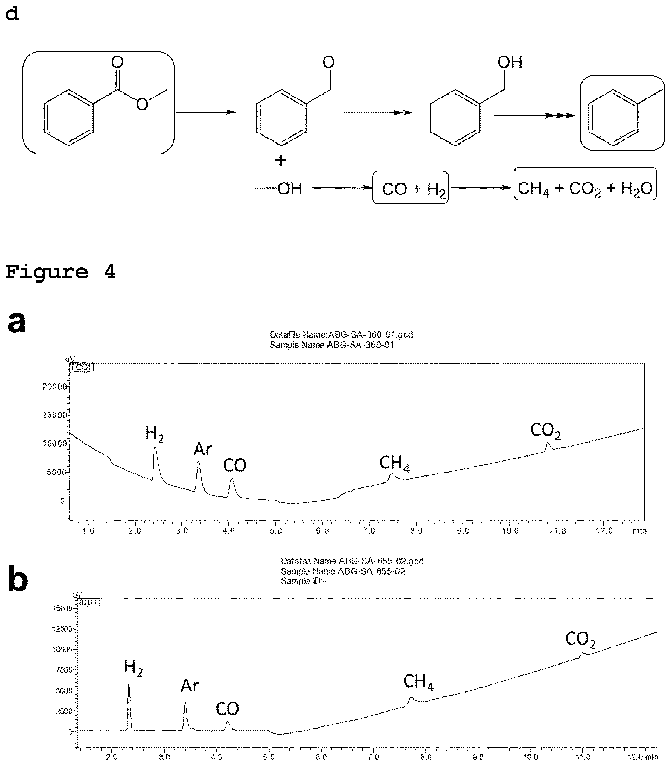

**Figure 3****:** Magnetocatalytic hydrodeoxygenation of methyl benzoate (**1**) with ICNPs. a) Solvent screening for 2 h reactions; b) Time profile; c) Time profile recorded while regularly switching ON and OFF the ACMF power supply; d) Proposed mechanism, with rectangles highlighting the observed compounds. Reaction conditions: methyl benzoate **1** (44.9 mg, 0.33 mmol), ICNPs (10 mg, 0.125 mmol of Fe), solvent (0.5 mL), H₂ (3 bar), magnetic field (µ₀Hₘₐₓ = 70 mT, 350 kHz). Product yields determined by GC-FID using tetradecane as the internal standard. Product selectivity is >99% in all cases. Data point are average values of three experiments. Global temperatures were determined by IR camera.
**Figure 4****:** GC-Headspace, gas phase analysis: a) after reaction of 4 h with methyl benzoate (0.33 mmol) under standard condition, b) of MeOH (0.33 mmol) at 1 h.

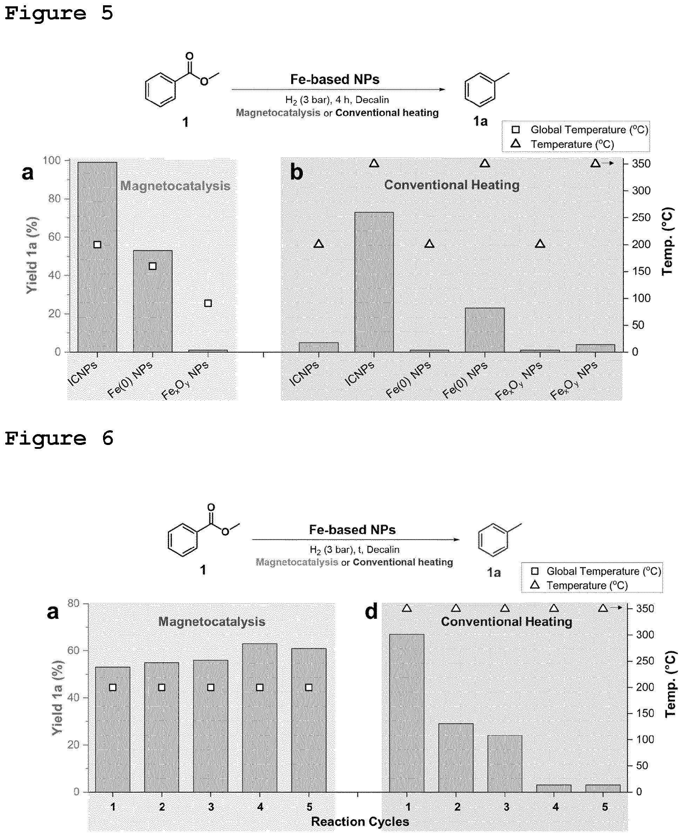

**Figure 5****:** Hydrodeoxygenation of methyl benzoate (**1**) with different Fe-based NPs using a) magnetocatalysis (350 kHz, 70 mT); and b) conventional heating at 200 °C and 350 °C. Reaction conditions: methyl benzoate **1** (44.9 mg, 0.33 mmol), Fe-based NPs (10 mg, 0.125 mmol of Fe), decalin (0.5 mL), H₂ (3 bar), ACMF or conventional heating, 4 h. Product yields determined by GC-FID using tetradecane as the internal standard. Product selectivity is >99% in all cases. Data point are average values of three experiments and error bars represent standard deviations. Global temperatures were determined by IR camera.

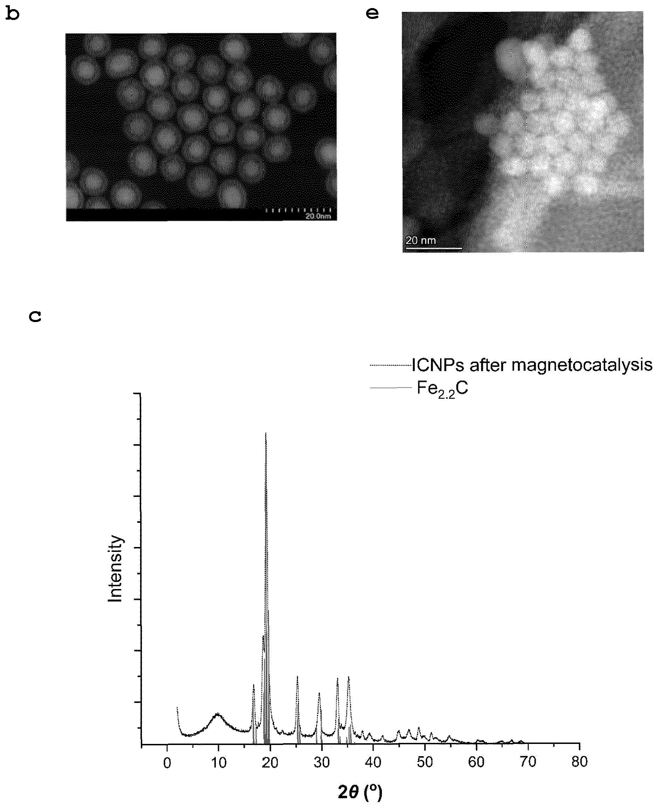

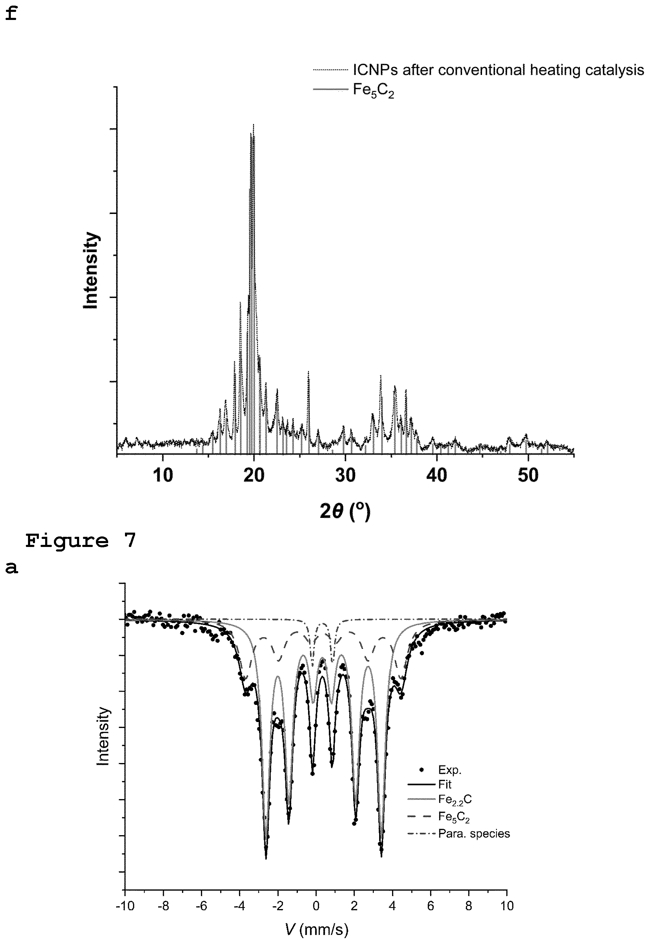

**Figure 6****:** Investigation of the reusability and stability of ICNPs for the hydrodeoxygenation of methyl benzoate (**1**) using magnetocatalysis (350 kHz, 70 mT) and conventional heating at 350 °C. a) Recycling experiment under magnetocatalytic conditions and characterization of ICNPs after 5 cycles by b) STEM-HAADF and c) PXRD; d) Recycling experiment under conventional heating at 350 °C and characterization of ICNPs after 5 cycles by e) STEM-HAADF and f) PXRD. Reaction conditions: methyl benzoate **1** (44.9 mg, 0.33 mmol), ICNPs (10 mg, 0.125 mmol of Fe), decalin (0.5 mL), H₂ (3 bar), ACMF or conventional heating, 2 h. Product yields determined by GC-FID using tetradecane as the internal standard. Product selectivity is >99% in all cases. Global temperatures were determined by IR camera.
**Figure 7****:** Low temperature (4 K) Mössbauer study of iron carbide nanoparticles after 5 cycles of methyl benzoate hydrodeoxygenation to toluene. (a) Mössbauer spectrum, (b) fitting parameters and resulting nanoparticles composition.
**Figure 8****:** Characterization of ICNPs nanoparticles after 5 cycles of methyl benzoate hydrodeoxygenation via XPS of Fe 2p, C 1s.
**Figure 9****:** Characterization of ICNPs nanoparticles after 5 cycles of methyl benzoate hydrodeoxygenation via VSM at 300 K and 5 K.
**Figure 10****:** TEM/SEM analysis of Fe(0) nanoparticles (11.3 ± 1.27 nm).
**Figure 11****:** Hydrodeoxygenation of substrates 1 (methyl benzoate, 0.33 mmol), 1b (benzaldehyde, 0.33 mmol), and 1c (benzyl alcohol, 1c) with ICNPs (10 mg, 0.125 mmol of Fe), decalin (0.5 mL), H₂ (3 bar), magnetic field (µ0Hmax=70 mT, 350 kHz). Product yields determined by GC-FID using tetradecane as the internal standard. Product selectivity is >99% in all cases.

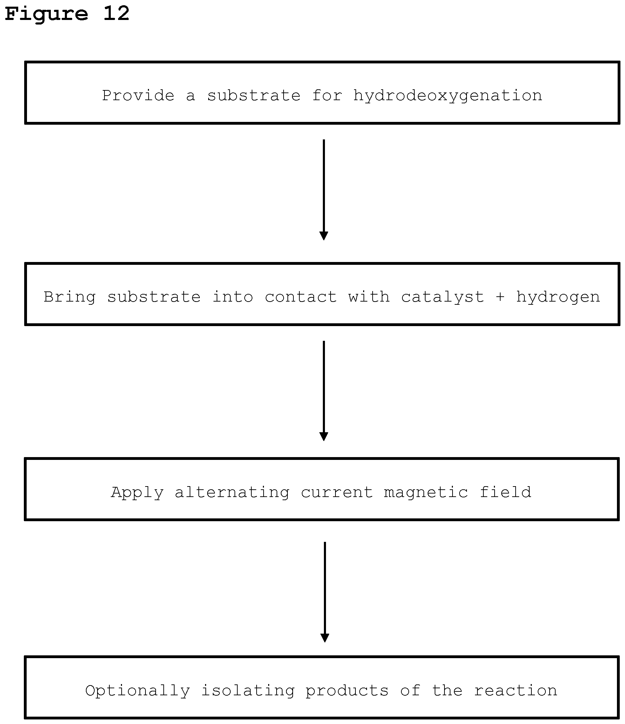

**Figure 12****:** Simplified overview of a method according to the present invention.

### Detailed Description

Embodiments according to the present invention will now be described in more detail.

As used herein, the term "particles" refers to ensembles of atoms that can possess various shapes and structures, which generally have a size of 1 nm - 100 µm. Nanoparticles / magnetic nanoparticles according to the present invention can have a size of 5-200 nm, preferably 5-50 nm.

As used herein, the term "magnetic particles" refers to particles that heat upon exposure to external alternating current magnetic fields. Examples of such magnetic particles include iron carbide particles, metallic iron particles, iron oxide particles, and combinations thereof. A method of preparing iron carbide nanoparticles is outlined in US 2020/0047166 A1.

As used herein, the term "neat conditions" refers to a reaction that is carried out without the presence of any additional solvents.

As used herein, the term "hydrodeoxygenation" refers to a reaction in which (in the presence of hydrogen) oxygen is removed from oxygen-containing substrates, such as alcohols, aldehydes, ketones and esters.

As used herein, the term "alternating current magnetic field" refers to the magnetic field produced by an alternating current.

As used herein, the term "3d transition metal" refers to metals from the first transition series or 3d series, including Sc, Ti, V, Cr, Mn, Fe, Co, Ni, Cu and Zn. As used herein, the term "4d transition metal" refers to metals from the second transition series or 4d series, including Y, Zr, Nb, Mo, Tc, Ru, Rh, Pd, Ag and Cd. As used herein, the term "5d transition metal" refers to metals from the third transition series or 5d series, including La, Hf, Ta, W, Re, Os, Ir, Pt, Au and Hg. As used herein, the term "noble metal" refers to metals that have outstanding resistance to oxidation, including Rh, Ru, Pd, Pt, Ag, Re, Os, Ir and Au.

As used herein, the "viscosity" of the solvent can be measured on a capillary viscometer at 20 °C.

As used herein, "low pressures" generally refer to pressures of 5 bar or less. As used herein, "mild temperatures" generally refer to temperatures of 220 °C or less.

As used herein, "iron-containing" (nano)particles refers to (nano)particles that contain iron. Examples of such iron-containing (nano)particles include, but are not limited to, iron carbide nanoparticles, Fe(0) nanoparticles, and iron oxide (FeₓO_{y}) nanoparticles.

As used herein, a substrate comprising, e.g., at least one ester moiety (or group) refers to a substrate comprising at least one ester moiety represented by the following structure:

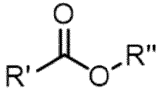

### Method according to the invention

According to an embodiment of the present invention, a method of hydrodeoxygenation of a substrate comprises:
bringing a substrate and hydrogen into contact with a catalyst; and
applying an alternating current magnetic field with a frequency of 30-600 kHz and a field amplitude of 10-150 mT;
wherein:
   the substrate comprises at least one ester moiety; and
   the catalyst comprises iron-containing particles, preferably wherein the iron-containing particles are nanoparticles.

According to a further embodiment of the present invention, a method of hydrodeoxygenation of a substrate comprises:
bringing a substrate, a solvent, and hydrogen into contact with a catalyst; and
applying an alternating current magnetic field with a frequency of 30-600 kHz and a field amplitude of 10-150 mT;
wherein:
   the catalyst comprises iron-containing particles, preferably wherein the iron-containing particles are nanoparticles;
   the substrate comprises at least one alcohol, aldehyde, ketone and/or ester group, preferably at least one alcohol, aldehyde and/or ester group, more preferably at least one ester group;
   the solvent has a boiling point of 180-300 °C, preferably wherein the solvent is decalin, dodecane, tetradecane or hexadecane; and
   the at least one alcohol, aldehyde, ketone and/or ester is reduced to the corresponding alkane.

The use of iron carbide nanoparticles in catalysis has been described by, *e.g.,* Chaudret B. et al (Angew. Chem. Int. Ed. 2019, 58, 11306-11310, "Hydrodeoxygenation Using Magnetic Inductions: High-Temperature Heterogeneous Catalysis in Solution") and in, *e.g.,* US2020/0047166 A1.

The various aspects of the method according to the present invention will be described in more detail in the following sections.

### Hydrodeoxygenation reaction

The method of the present invention is particularly suitable for a hydrodeoxygenation reaction, for example a hydrodeoxygenation of an alcohol substrate, an aldehyde substrate, a ketone substrate, and/or an ester substrate (*i.e.,* a substrate containing at least one alcohol, aldehyde, ketone and/or ester moiety). Preferably the hydrodeoxygenation reaction of the substrate results in the corresponding alkane.

According to a preferred embodiment, the hydrodeoxygenation reaction is a hydrodeoxygenation reaction of an ester substrate. Preferably the hydrodeoxygenation reaction of the ester substrate results in the corresponding alkane.

The method and the catalyst according to the present invention, allow the hydrodeoxygenation of the substrate, preferably an ester substrate, to its corresponding alkane. It is thus preferred, that the hydrodeoxygenation reaction is a hydrodeoxygenation reaction of an ester moiety to the corresponding alkane moiety. The method according to an embodiment of the present invention can thus be summarised as follows:

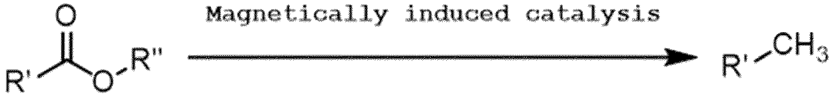

### Substrate

The substrates according to the present invention are not particularly limited, except that the substrate should include a moiety that can undergo a hydrodeoxygenation reaction. Examples of suitable substrates include but are not limited to substrates comprising at least one alcohol, aldehyde, ketone, and/or ester.

According to a preferred embodiment, the substrate is an ester substrate, *i.e.,* a substrate containing at least one ester moiety/group. Such an ester moiety can be represented by the following structure:

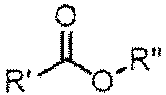

The definition of R' and R" is not particularly limited, but can include, for example:
R' is C₆H₁₁, phenyl, napththyl, biphenyl, furyl, a linear or branched alkyl group containing 1-20 carbon atoms, or cyclohexyl,
   wherein the phenyl, napththyl, biphenyl, furyl, a linear or branched alkyl group containing 1-20 carbon atoms, or cyclohexyl is optionally substituted with an amide, an amine, an alkyl, an ether, an ester, an alcohol, an aldehyde, a ketone, a halogen,
   preferably wherein R' is C₆H₁₁, phenyl or furyl, wherein the phenyl is optionally substituted with -N(CH₃)₂, -NH₂, -CN, -OCH₃, -NO₂, -CHO, -OH, -I, -CF₃, -Cl, -Br, -F, -CH₃, - C(O)OCH₃, and phenyl, and the furyl is optionally substituted with -C(O)OCH₃;
   R" is a linear or branched alkyl group containing 1-20 carbon atoms, preferably 1-10 carbon atoms, -CH₂(C₆H₅), phenyl, furyl, benzyl, an ester, an ether, a ketone, an aldehyde or an alcohol;
      preferably wherein R" is a linear or branched alkyl group containing 1-20 carbon atoms, optionally substituted with phenyl, furyl, benzyl, an ester, an ether, a ketone, an aldehyde or an alcohol.

According to a preferred embodiment:
R' is C₆H₁₁, phenyl or furyl, wherein the phenyl is optionally substituted with -N(CH₃)₂, -NH₂, -CN, -OCH₃, -NO₂, -CHO, -OH, -I, -CF₃, -Cl, -Br, -F, -CH₃, -C(O)OCH₃, and phenyl, and the furyl is optionally substituted with - C(O)OCH₃;
R" is a linear or branched alkyl group containing 1-20 carbon atoms, optionally substituted with phenyl, furyl, benzyl, an ester, an ether, a ketone, an aldehyde or an alcohol.

In a preferred embodiment of the presently claimed method, the ester substrate undergoes hydrodeoxygenation to the corresponding alkane substrate. That is, the ester is reduced to the corresponding alkane.

In an embodiment of the present invention, the substrate is:

| | |
|---|---|
| A | 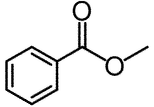 |
| B | 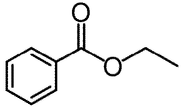 |
| C | 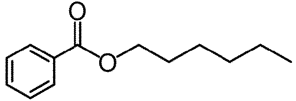 |
| D | 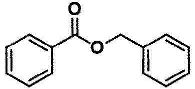 |
| E | 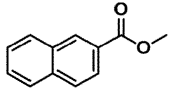 |
| F | 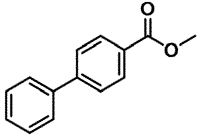 |
| G | 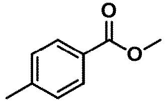 |
| H | 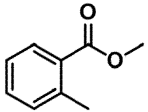 |
| I | 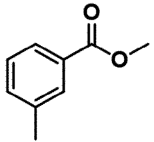 |
| J | 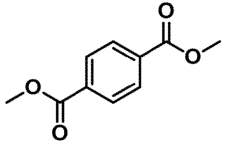 |
| K | 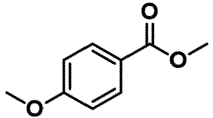 |
| L | 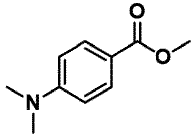 |
| M | 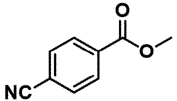 |
| N | 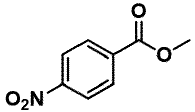 |
| O | 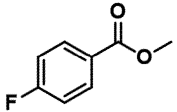 |
| P | 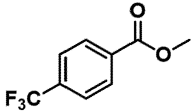 |
| Q | 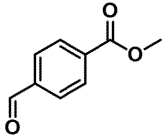 |
| R | 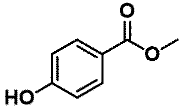 |
| S | 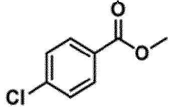 |
| T | 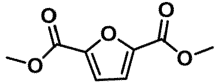 |
| U | 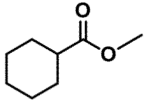 |
| V | 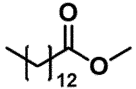 |

### Hydrogen

According to a preferred embodiment of the present invention, the pressure of the hydrogen is 1-100 bar, preferably 1-20 bar, more preferably 1-10 bar, even more preferably 1-5 bar, most preferably 2-4 bar.

### Magnetic field

The method of hydrodeoxygenation of a substrate includes the application of a magnetic field. It is preferred that an alternating current magnetic field (ACMF) is applied.

The magnetic field has a frequency and field amplitude sufficient to effect the hydrodeoxygenation reaction.

According to a preferred embodiment, the magnetic field heats the magnetic (nano)particles to a temperature of 100-450 °C, preferably 150-400 °C, more preferably 150-350 °C.

According to a preferred embodiment, the magnetic field heats the iron carbide nanoparticles to a temperature of 100-450 °C, preferably 250-400 °C, more preferably 300-350 °C.

It is preferred that the applied magnetic field has a field amplitude of 10 - 150 mT, preferably 15 - 150 mT, more preferably 50 - 100 mT, even more preferably 65 - 90 mT, even more preferably 65 - 75 mT, most preferably 70 mT.

It is also preferred that the applied magnetic field has a frequency of 30 - 600 kHz, preferably 150-450 kHz, more preferably 250-400 kHz, even more preferably 300-400 kHz, most preferably 350 kHz.

According to a preferred embodiment, an alternating current magnetic field with a frequency of 250 - 400 kHz and a field amplitude of 65 - 90 mT is applied. According to a more preferred embodiment, an alternating current magnetic field with a frequency of 300 - 400 kHz and a field amplitude of 65 - 75 mT is applied.

The advantage of applying such a magnetic field is achieving good selectivity and yields in the hydrodeoxygenation reaction, while providing localised, rapid and energy efficient heating of the catalyst. A further advantage is that the reaction can proceed at low pressure and mild temperature.

### Catalyst components

The catalyst according to the present invention contains magnetic particles. Examples of such magnetic particles include iron-containing particles, such as iron carbide nanoparticles and Fe(0) nanoparticles.

According to a preferred embodiment, the magnetic particles have a size of 5-200 nm, preferably 5-50 nm, most preferably 10-20 nm.

It is preferred that the magnetic particles are magnetic nanoparticles. This has the advantage that the nanoparticles provide a more localised and efficient heating.

According to the present invention, the magnetic particles comprise iron. For example, the magnetic particles are iron-containing particles that can comprise iron carbides, iron phosphides, iron sulphides, Fe(0) or iron oxides.

The catalyst according to the present invention comprises iron-containing particles, preferably iron-containing nanoparticles. Preferably, the iron-containing particles/nanoparticles include:
i) iron phosphide nanoparticles;
ii) iron sulphide nanoparticles;
iii) iron carbide nanoparticles; and/or
iv) Fe(0) nanoparticles.

More preferably, the iron-containing particles/nanoparticles include iron carbide nanoparticles and/or Fe(0) nanoparticles.

It is preferred that the catalyst according to the present invention comprises iron carbide nanoparticles. Experiments demonstrated that iron carbide nanoparticles are more active than metallic iron and iron oxides for hydrodeoxygenation reactions. Furthermore, iron carbide nanoparticles are found to be very stable when used in magnetocatalysis, which stands in contrast to their rapid degradation when used under conventional thermal catalysis.

It is preferred that the catalysts comprises iron carbide nanoparticles. According to an embodiment of the invention, when the catalyst comprises iron carbide nanoparticles, it is preferred that 40-100%, more preferably 70-90% of the iron carbide nanoparticles are present in an Fe_{2.2}C crystalline structure. The remainder of the iron atoms can be present as, *e.g*., Fe₅C₂, Fe(0), and FeₓO_{y}.

Preferably, the iron carbide nanoparticles comprise Fe_{2.2}C, optionally wherein the iron carbide nanoparticles further comprise Fe₅C₂.

According to a preferred embodiment, when the catalyst comprises iron carbide nanoparticles, the iron carbide nanoparticles consist of Fe_{2.2}C and Fe₅C₂, optionally wherein the ratio of Fe_{2.2}C to Fe₅C₂ (Fe_{2.2}C : Fe₅C₂), is 40-99 : 1-60, preferably 50-99 : 1-50, more preferably 60-99 : 1-40. According to a preferred embodiment, when the catalyst comprises iron carbide nanoparticles, the iron carbide nanoparticles consist of Fe_{2.2}C.

It has been found that working within the above ranges, improves the magnetic heating performance.

According to a preferred embodiment, the catalyst consists of iron-containing nanoparticles. Iron-containing nanoparticles can be, for example, iron carbide nanoparticles, iron phosphide nanoparticles, iron sulphide nanoparticles, Fe(0) nanoparticles and iron oxide nanoparticles.

It is preferred that the catalyst consists of iron-containing nanoparticles, wherein the iron-containing nanoparticles comprise iron carbide nanoparticles and/or Fe(0) nanoparticles. It is more preferred that the catalyst consists of iron-containing nanoparticles, wherein the iron-containing nanoparticles include iron carbide nanoparticles.

According to a preferred embodiment, the catalyst consists of iron-containing nanoparticles, wherein the iron-containing nanoparticles comprise:
50-100 wt%, preferably 80-100 wt%, of iron carbide nanoparticles and/or Fe(0) nanoparticles, preferably iron carbide nanoparticles;
0-50 wt%, preferably 0-20 wt%, of iron oxide nanoparticles. The catalyst can further contain other iron-containing nanoparticles, such as iron-containing paramagnetic species. Preferably, only 0-10 wt%, more preferably 0-5 wt%, of these other iron-containing nanoparticles are present.

According to a preferred embodiment, the catalyst consists of iron carbide nanoparticles and/or Fe(0) nanoparticles, preferably wherein the catalyst consists of iron carbide nanoparticles.

According to a preferred embodiment, the catalyst does not contain any noble metals.

According to another preferred embodiment, the catalyst does not contain any 3d, 4d and/or 5d transition metals, other than iron. It is most preferred that the catalyst does not contain any 3d, 4d and 5d transition metals, other than iron.

### General

According to an embodiment of the present invention, the hydrodeoxygenation reaction is carried out under neat conditions. This can be particularly useful when the substrate is a liquid.

According to another embodiment of the present invention, the reaction is carried out in a solvent. It is preferred that the solvent has a boiling point of 80-350 °C, preferably 120-340 °C, more preferably 160-330 °C, even more preferably 170-320 °C, most preferably 180-300 °C. Working within these ranges has the advantage that the activity of the reaction is improved. Without wishing to be bound by theory, boiling points that are too low can be problematic due to the Leidenfrost effect. The solvent can heavily boil around the catalyst, building an isolating gaseous layer and decreasing the activity. It is thus advantageous to work within the above-mentioned temperature ranges.

It is further preferred that the solvent has a viscosity of 0.1-5 mPa·s, preferably 0.5-3 mPa·s. Working within these ranges has the advantage that the reaction rate is improved. Working within these ranges can further prevent mass transfer limitations from the substrate to the catalytically active sites. If the solvent is too viscous, the reaction rate can decrease because the substrate molecules take longer to diffuse and reach the catalyst.

It is particularly preferred that the solvent has a boiling point of 180 - 300 °C and a viscosity of 0.5-3 mPa·s.

According to a preferred embodiment, the solvent is a hydrocarbon-based solvent. Preferably, the hydrocarbon-based solvent is a linear or branched C₇-C₂₀ hydrocarbon-based solvent. Examples of suitable solvents include but are not limited to tert-butanol, dichloroethane, DMF, DMSO, dioxane, xylenes, an ionic liquid such as an imidazolium-based ionic liquid or a phosphonium-based liquid, supercritical CO₂, 2-methyl THF, decalin, dodecane, tetradecane, and hexadecane. It is preferable that the solvent is decalin, dodecane, tetradecane, or hexadecane.

If a solvent is used, the substrate and/or the catalyst can be dispersed in the solvent.

Advantages of the catalysts described in the sections above, include their recyclability and re-usability. Furthermore, their use in the described methods are particularly advantageous in providing the hydrodeoxygenation of, e.g., esters to the corresponding alkanes with good yields, good selectivity and good substrate scope, whilst the catalyst is stable and recyclable. Furthermore, the hydrodeoxygenation reaction proceeds under mild reaction conditions (low pressures and temperatures), is efficient and sustainable. The method also provides for a potential adaptivity of the catalytic process to intermittent power supply.

The catalysts described above can be used in a hydrodeoxygenation reaction, such as a hydrodeoxygenation reaction of an alcohol, an aldehyde, a ketone and/or an ester. Preferably they are used in a hydrodeoxygenation reaction of an ester, preferably wherein the ester is reduced to the corresponding alkane.

The catalysts described above can also be used in any of the hydrodeoxygenation reactions described above.

Overall, according to a most preferred embodiment, the invention is directed towards a method of hydrodeoxygenation of a substrate, wherein the method comprises:
bringing a substrate, hydrogen and a solvent into contact with a catalyst; and
applying an alternating current magnetic field with a frequency of 300-400 kHz and a field amplitude of 65-75 mT;
wherein:
   the substrate comprises at least one alcohol, aldehyde, ketone and/or ester moiety, preferably at least one ester moiety;
   the solvent has a boiling point of 180-300 °C, preferably wherein the solvent is decalin, dodecane, tetradecane or hexadecane;
   the hydrogen is pressured at 1-5 bar; and
   the catalyst consists of iron-containing nanoparticles, comprising iron carbide nanoparticles.

Preferably, the iron carbide nanoparticles comprise Fe_{2.2}C, optionally wherein the iron carbide nanoparticles further comprise Fe₅C₂. Preferably, the iron carbide nanoparticles consist of Fe_{2.2}C and Fe₅C₂, optionally wherein the ratio of Fe_{2.2}C to Fe₅C₂, is 40-99 : 1-60, preferably 50-99 : 1-50, more preferably 60-99 : 1-40.

It is preferred that the iron-containing nanoparticles comprise 80-100 wt% of iron carbide nanoparticles, 0-20 wt% of iron oxide nanoparticles. Further iron-containing nanoparticles can be present to a maximum of 5 wt%.

In this most preferred embodiment, it is preferable that the ester is reduced to the corresponding alkane. Furthermore, it is preferred that the catalyst does not contain any noble metals.

Such a method is furthermore particularly advantageous in providing the hydrodeoxygenation of esters to the corresponding alkanes with good yields, good selectivity and good substrate scope, whilst the catalyst is stable and recyclable. Furthermore, the hydrodeoxygenation reaction proceeds under mild reaction conditions (low pressures and temperatures), is efficient and sustainable. The method also provides for a potential adaptivity of the catalytic process to intermittent power supply.

### The invention includes:

*Item* 1: Method of hydrodeoxygenation of a substrate, wherein the method comprises:
   bringing a substrate and hydrogen into contact with a catalyst; and applying an alternating current magnetic field with a frequency of 30-600 kHz and a field amplitude of 10-150 mT; wherein:
   the substrate comprises at least one alcohol, aldehyde, ketone and/or ester moiety; preferably at least one alcohol, aldehyde and/or ester moiety; more preferably at least one ester moiety; and
   the catalyst comprises iron-containing particles, preferably wherein the iron-containing particles are nanoparticles.
*Item 2:* Method of hydrodeoxygenation of a substrate, wherein the method comprises:
   bringing a substrate, a solvent, and hydrogen into contact with a catalyst; and applying an alternating current magnetic field with a frequency of 30-600 kHz and a field amplitude of 10-150 mT; wherein:
   the catalyst comprises iron-containing particles, preferably wherein the iron-containing particles are nanoparticles;
   the substrate comprises at least one alcohol, aldehyde, ketone and/or ester group, preferably at least one alcohol, aldehyde and/or ester group, more preferably at least one ester group;
   the solvent has a boiling point of 180-300 °C, preferably wherein the solvent is decalin, dodecane, tetradecane or hexadecane; and
   the at least one alcohol, aldehyde, ketone and/or ester is reduced to the corresponding alkane.
*Item 3:* Method of hydrodeoxygenation according to any one of items 1-2, wherein the iron-containing particles comprise:
   i) iron phosphide nanoparticles;
   ii) iron sulphide nanoparticles;
   iii) iron carbide nanoparticles; and/or
   iv) Fe(0) nanoparticles;
   preferably wherein the iron-containing particles comprise iron carbide nanoparticles and/or Fe(0) nanoparticles.
*Item 4:* Method of hydrodeoxygenation according to any one of items 1-3, wherein the catalyst does not contain any noble metals.
*Item 5:* Method of hydrodeoxygenation according to any one of items 1-4, wherein the iron-containing particles comprise iron carbide nanoparticles, and wherein the iron carbide nanoparticles comprise Fe_{2.2}C, optionally wherein the iron carbide nanoparticles further comprise Fe₅C₂.
*Item* 6: Method of hydrodeoxygenation according to any one of items 1-5, wherein the iron-containing particles comprise iron carbide nanoparticles, wherein the iron carbide nanoparticles consist of: Fe_{2.2}C and Fe₅C₂, optionally wherein the ratio of Fe_{2.2}C to Fe₅C₂, is 40-99 : 1-60, preferably 50-99 : 1-50, more preferably 60-99 : 1-40; or Fe_{2.2}C.
*Item* 7: Method of hydrodeoxygenation according to any one of items 1-6, wherein the catalyst consists of iron-containing nanoparticles, preferably wherein:
   i) the iron-containing nanoparticles comprise iron carbide nanoparticles and/or Fe(0) nanoparticles, preferably iron carbide nanoparticles;
   ii) the iron-containing nanoparticles comprise:
      50-100 wt%, preferably 80-100 wt%, of iron carbide nanoparticles and/or Fe(0) nanoparticles, preferably iron carbide nanoparticles, and
      0-50 wt%, preferably 0-20 wt% of iron oxide nanoparticles.
*Item* 8: Method according to any one of items 1-7, wherein the catalyst consists of iron-containing nanoparticles, wherein the iron-containing nanoparticles consist of iron carbide nanoparticles and/or Fe(0) nanoparticles, preferably iron carbide nanoparticles.
*Item 9:* Method according to any one of items 1-8, wherein:
   the field amplitude is 15-150 mT, preferably 50-100 mT, more preferably 65-90 mT, even more preferably 65-75 mT, most preferably 70 mT; and/or
   the frequency is 150-450 kHz, preferably 250-400 kHz, more preferably 300-400 kHz, most preferably 350 kHz;
   preferably wherein the field amplitude is 65-75 mT and the frequency is 300-400 kHz.
*Item* 10: Method according to any one of items 1-9, wherein the substrate is represented by the following formula (I): 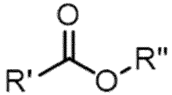 wherein:
   R' is C₆H₁₁, phenyl, napththyl, biphenyl, furyl, a linear or branched alkyl group containing 1-20 carbon atoms, or cyclohexyl,
      wherein the phenyl, napththyl, biphenyl, furyl, a linear or branched alkyl group containing 1-20 carbon atoms, or cyclohexyl is optionally substituted with an amide, an amine, an alkyl, an ether, an ester, an alcohol, an aldehyde, a ketone, a halogen,
      preferably wherein R' is C₆H₁₁, phenyl or furyl, wherein the phenyl is optionally substituted with -N(CH₃)₂, -NH₂, -CN, -OCH₃, -NO₂, -CHO, -OH, - I, -CF₃, -Cl, -Br, -F, -CH₃, -C(O)OCH₃, and phenyl, and the furyl is optionally substituted with - C(O)OCH₃;
   R" is a linear or branched alkyl group containing 1-20 carbon atoms, preferably 1-10 carbon atoms; - CH₂(C₆H₅), phenyl, furyl, benzyl, an ester, an ether, a ketone, an aldehyde or an alcohol;
      preferably wherein R" is a linear or branched alkyl group containing 1-20 carbon atoms, optionally substituted with phenyl, furyl, benzyl, an ester, an ether, a ketone, an aldehyde or an alcohol.
*Item* 11: Method according to any one of items 1-10, wherein the hydrogen is pressurised at 1-100 bar, preferably 1-20 bar, more preferably 1-10 bar, even more preferably 1-5 bar, most preferably 2-4 bar.
*Item 12:* Method according to any one of items 1-11, wherein the alcohol, aldehyde, ketone and/or ester are reduced to the corresponding alkane.
*Item 13:* Method according to any one of items 1-12, wherein the hydrodeoxygenation is carried out in a solvent, preferably wherein:
   i) the solvent has a boiling point of 80-350 °C, preferably 120-340 °C, more preferably 160-330 °C, even more preferably 170-320 °C, most preferably 180-300 °C; and/or
   ii) the solvent has a viscosity of 0.1-5 mPa·s, preferably 0.5-3 mPa·s.
*Item 14:* Method according to any one of items 1-13, wherein the solvent is a hydrocarbon-based solvent; preferably wherein the hydrocarbon-based solvent is a linear or branched C₇-C₂₀ hydrocarbon-based solvent; more preferably wherein the solvent is tert-butanol, dichloroethane, DMF, DMSO, dioxane, xylenes, an ionic liquid such as an imidazolium-based ionic liquid or a phosphonium-based liquid, supercritical CO₂, 2-methyl THF, decalin, dodecane, tetradecane, and hexadecane; most preferably wherein the solvent is decalin, dodecane, tetradecane or hexadecane.
*Item 15:* Method according to any one of items 1-14, wherein the magnetic field heats:
   the iron carbide and/or Fe(0) nanoparticles to a temperature of 100-450 °C, preferably 150-400 °C, most preferably 150-350 °C; and/or
   the iron carbide nanoparticles to a temperature of 100-450 °C, preferably 250-400 °C, most preferably 300-350 °C.
*Item 16:* Use of a catalyst in a hydrodeoxygenation reaction, wherein the hydrodeoxygenation reaction involves:
   bringing a substrate, a solvent, and hydrogen into contact with the catalyst; and applying an alternating current magnetic field with a frequency of 30-600 kHz and a field amplitude of 10-150 mT, preferably a frequency of 300-400 kHz and a field amplitude of 65-75 mT; wherein:
   the catalyst comprises iron-containing nanoparticles, wherein the iron-containing nanoparticles comprise iron carbide nanoparticles and/or Fe(0) nanoparticles;
   the substrate comprises at least one alcohol, aldehyde, ketone and/or ester group, preferably at least one alcohol, aldehyde and/or ester, preferably an ester;
   the solvent has a boiling point of 180-300 °C, preferably wherein the solvent is decalin, dodecane, tetradecane or hexadecane;
   the at least one alcohol, aldehyde, ketone and/or ester is reduced to the corresponding alkane.
*Item* 17: Use of a catalyst in a hydrodeoxygenation reaction according to item 16, wherein:
   the catalyst consists of iron-containing nanoparticles; and/or
   the catalyst does not contain any noble metals; and/or
   the iron-containing nanoparticles comprise iron carbide nanoparticles comprising Fe_{2.2}C and optionally Fe₅C₂; and/or
   the iron-containing nanoparticles comprise iron carbide nanoparticles and the iron carbide nanoparticles consist of Fe_{2.2}C and Fe₅C₂, optionally wherein the ratio of Fe_{2.2}C to Fe₅C₂, is 40-99 : 1-60, preferably 50-99 : 1-50, more preferably 60-99 : 1-40.

### Examples

Examples according to the present invention will be presented.

### General:

All syntheses were performed under argon either by using Schlenk techniques or in a glove box. Solvents were purified through a solvent purification system (MBraun-SPS-7) or dried over activated 4 Å molecular sieves then degassed and preserved under an argon atmosphere before use. Hexadecylamine (HDA, 99%), palmitic acid (PA, 99%) and FeCl₂ anhydrous (98%) were purchased from Sigma-Aldrich and abcr. CO and H₂ gas were purchased from Air Liquide. Esters for the substrate scope are purchased from the local suppliers (e.g. Sigma-Aldrich, abcr, Alfa Aesar) and used without further purification.

### Characterisation techniques:

All XPS samples were prepared within an Argon-filled glovebox. Subsequently, these samples were carefully transferred to a mobile analysis chamber inside the glovebox, which was then connected to the XPS devices. The X-ray photoelectron spectroscopy (XPS) experiments were recorded using monochromatized Al-Kα (1487 eV) radiation and a Phoibos NAP-150 hemispherical analyzer from SPECS GmbH. High resolution spectra were collected using 20 eV pass energy. The instrument work function was calibrated to give an Au 4f7/2 metallic gold binding energy of 83.96 eV. Instrument base pressure was 5 * 10-8 mbar. TEM images were recorded on a Hitachi HF2000 operating at 200 kV, and STEM-HAADF was performed on a HITACHI HD-2700 cold FEG operated at 200 kV at the shared facility with the Max Planck Institute for Coal research (KoFo). Samples for electron microscopy were prepared by depositing the powder onto a copper grid with an amorphous carbon support film. To determine the size of the nanoparticles, the particles were measured using ImageJ with a count of at least 200 nanoparticles. The Gaussian fit of the frequency of size ranges gave the size distribution. For the ICP-MS measurement, the samples were digested with 8 mL HNO₃ in the CEM microwave MARS 6. The microwave program is as follows: Power: 1500 W, ramp up to 200 degrees for 30 min, hold time 15 min, temperature 200 °C. The samples were then measured using the Shimadzu ICPMS-2030 ICP-MS device.

Magnetic measurements were performed on a Quantum Device PPMS Evercool. About 10 mg of the sample was placed in a sealed teflon capsule in the glovebox. magnetization versus magnetic field measurements (hysteresis loop) were conducted at 300 and/or 5 K, with an external field of up to ±3 T. The 57Fe Mössbauer measurement was measured in-house by Derya Demirbas. In the glovebox, an iron material was loaded into a teflon sample holder (ca. 1 cm * 1 cm), and if needed, mixed with icosane or boron nitride to fill any additional space. The 57Fe Mössbauer spectra were collected on a spectrometer with conventional alternating constant acceleration of the γ-ray source. The sample temperature was controlled using an Oxford Instrument Variox for zero field measurements or a Cryogen-Free Magnet (CFM) with integrated variable temperature insert (VTI) perpendicular to the γ beam. The 57Co source in Rh matrix (1.8 GBq) rests at room temperature in the gap of the magnet system at the zero field position, by using a reentrant bore. Isomer shifts are quoted relative to α-iron at 300 K. The 57Fe Mössbauer were simulated and fitted with MX program written by Dr. Eckhard Bill.

### Product analysis:

Product analysis was done by GC-FID (gas chromatography coupled with flame ionization detection) on a Shimadzu GC 2030 equipped with a CP-WAX-52CB. GC-MS (gas chromatography coupled with mass spectrometry) on a Shimadzu QP 2020 instrument used with its internal compound library for product identification and identification of unknown products. Product peak area to the peak area of tetradecane standard were used for product quantification for GC-FID.

### Catalytic experiments:

*Magneto-catalytic experiments (magnetic induction heating)* Typically for all magneto-catalytic experiments, iron carbide nanoparticles (10.0 mg, 0.125 mmol Fe), solvent (0.5 mL), and the substrate (0.33 mmol) were placed in a FP bottle. The FP bottle was degassed, and pressurized with the desired pressure of hydrogen (3 bar). The reaction mixture was placed at the center of a copper coil at the desired magnetic field amplitude and fixed frequency of 350 kHz. Once the reaction was finished, the reactor was cooled and vented. After filtration, the reaction mixture was analysed by GC-FID using tetradecane as the internal standard.

### Catalysis with conventional heating

For conventional heating system of 200 °C, the catalyst, solvent (0.5 mL), and substrate (0.10 mmol) were placed in a FP bottle. The FP bottle was degassed, and pressurized with the desired pressure of hydrogen (3 bar). The reaction mixture placed in an oil bath and the reaction performed at the desired temperature. Once the reaction was finished, the FP bottle was cooled and vented. After filtration, the reaction mixture was analyzed by GC-FID using tetradecane as the internal standard.

For reactions performed at higher temperatures (350 °C), stainless steel autoclaves heated in aluminum heating blocks were used. Once the reaction was finished, the FP bottle was cooled and vented. After filtration, the reaction mixture was analyzed by GC-FID using tetradecane as the internal standard.

### Kinetic study

Time profiles were collected following similar protocols, with individual reactions for each selected time.

### Recycling experiments

Iron carbide nanoparticles (10.0 mg, 0.125 mmol Fe), decalin (0.5 mL), and methyl benzoate (44.9 mg, 0.33 mmol) were placed in a FP bottle. The FP bottle was degassed, and pressurized with the desired pressure of hydrogen (3 bar). The reaction mixture was placed at the center of a copper coil at the desired magnetic field amplitude of 70 mT and fixed frequency of 350 kHz for 2 h. Once the reaction was finished, the reactor was cooled and vented. After filtration, the reaction mixture was analyzed by GC-FID using tetradecane as the internal standard. For the next cycle, fresh portions of the methyl benzoate (44.9 mg, 0.33 mmol) and decalin (0.5 mL) were added and the reaction mixture was performed again. This procedure was repeated for each catalyst cycle by pressurizing the Fisher-Porter bottle with 3 bar of hydrogen.

Following a similar protocol, for the conventional heating recycling experiments at 350 °C, iron carbide nanoparticles (10.0 mg, 0.125 mmol Fe), decalin (0.5 mL), and methyl benzoate (44.9 mg, 0.33 mmol) were placed in a stainless-steel autoclave. The reaction mixture was placed at aluminum heating blocks at 350 °C for 2 h. Once the reaction was finished, the reactor was cooled and vented. After filtration, the reaction mixture was analyzed by GC-FID using tetradecane as the internal standard. For the next cycle, fresh portions of the methyl benzoate (44.9 mg, 0.33 mmol) and decalin (0.5 mL) were added and the reaction mixture was performed again. This procedure was repeated for each catalyst cycle by pressurizing the Fisher-Porter bottle with 3 bar of hydrogen.

### Example 1: Synthesis of Fe(0) nanoparticles (Fe(0) NPs) and synthesis of iron carbide nanoparticles (ICNPs).

The nanoparticles were prepared following a previously reported procedure (Bordet, A. et al. Magnetically induced continuous CO2 hydrogenation using composite iron carbide nanoparticles of exceptionally high heating power. Angewandte Chemie International Edition 55, 15894-15898 (2016)).

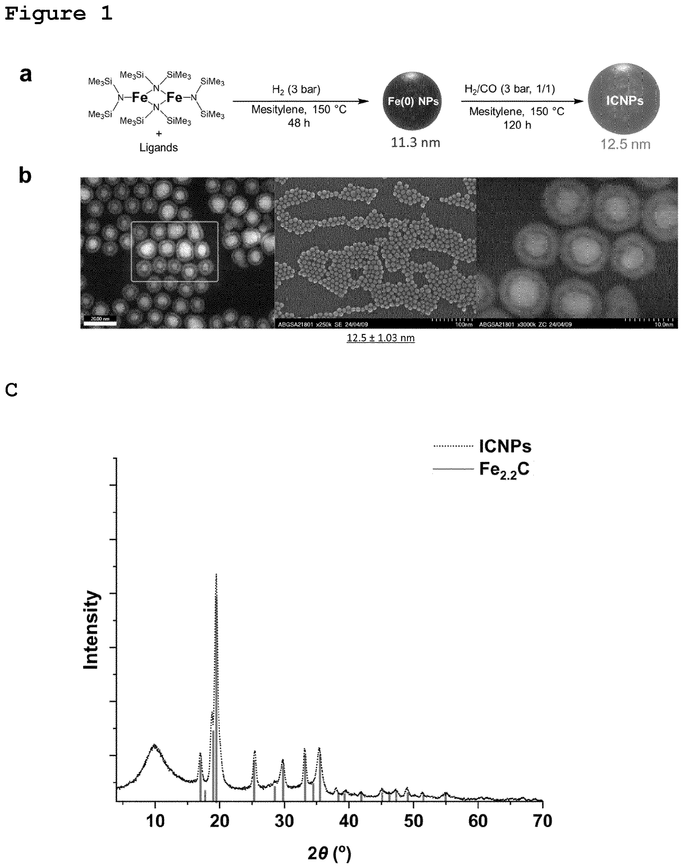

The iron carbide nanoparticles were obtained in two steps through the carbidisation of preformed Fe(0)nanoparticles (see Figure 1a).

### Fe(0) nanoparticles

{Fe[N(SiMes₃)₂]₂}₂ was prepared according to the reported protocol outlined in Broere, D. l. L., Čorić, I., Brosnahan, A. & Holland, P. L. Quantitation of the THF Content in Fe [N (SiMe3) 2] 2· x THF. *Inorganic Chemistry* **56,** 3140-3143 (2017).

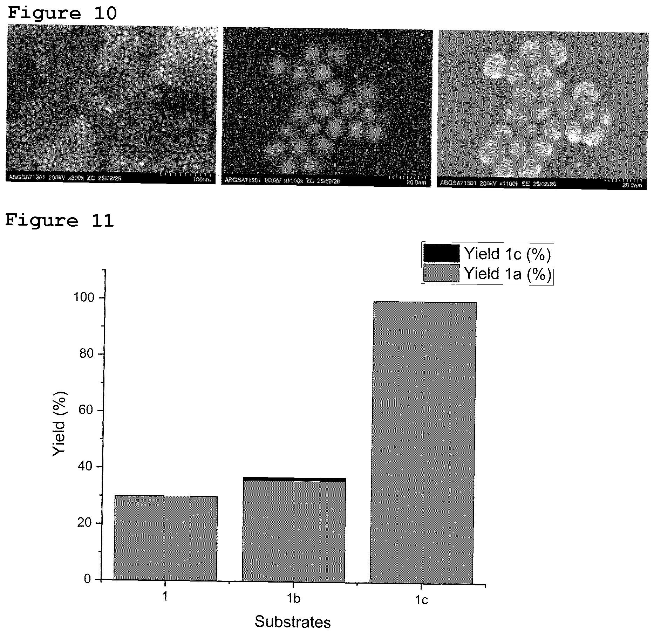

In a Fischer Porter bottle (stored inside the glove box), mixture of solutions that were added in order involve: {Fe[N(SiMes₃)₂]₂}₂ (1.0 mmol, 753 mg) that was dissolved in 7 mL mesitylene, Palmitic acid (PA, 1.3 mmol, 666.4 mg) dissolved in 15 mL mesitylene and hexadecylamine (HDA, 1.0 mmol, 483 mg) dissolved in 15 mL mesitylene respectively. The solution switches its color from green to yellowish and then to blackish color. The Fischer Porter bottle was pressurized with H₂ (3 bar) that was then followed by vigorous stirring and heating in oil bath at 150 °C for 48h. The nanoparticles are then recovered by decantation, which is assisted by a magnet and then by washing it 3 x 10 mL toluene, and 3 x 10 mL with THF. The resulting nanoparticles are dried under vacuum and stored in the glovebox. Following this method allows the production of size-controlled Fe(0) NPs of 11.3 nm (Figure 10).

### Carbidisation of Fe(0) nanoparticles to afford iron carbide nanoparticles

The Fe(0) nanoparticles (100 mg, 0.45 mmol of Fe) were dispersed in a Fischer Porter bottle containing 9 mL mesitylene that was stored in the glove box. The bottle was pressurized by CO / H₂ (2 bar/ 2 bar), followed by heating and stirring at 150 °C for 120 h. The obtained nanoparticles were recovered by decantation that are assisted by magnets, washed with 3 x 5 mL toluene, then dried under vacuum and stored in the glovebox. This allowed the production of iron carbide nanoparticles with very high heating power under ACMF (SAR of ca. 2500 W g⁻¹ at f = 100 kHz and µ₀*H*ₘₐₓ = 66 mT). The nanoparticles were 12.5 nm in size (see Figure 1b).

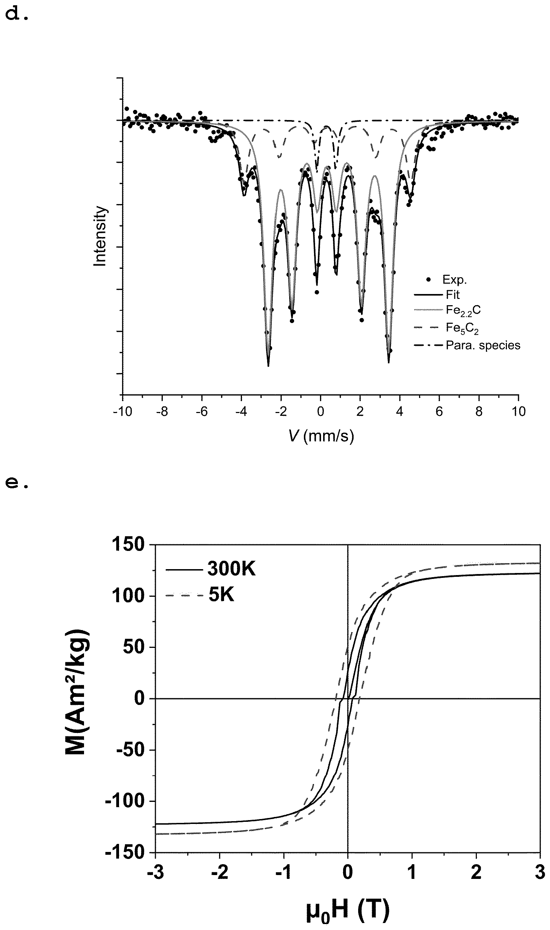

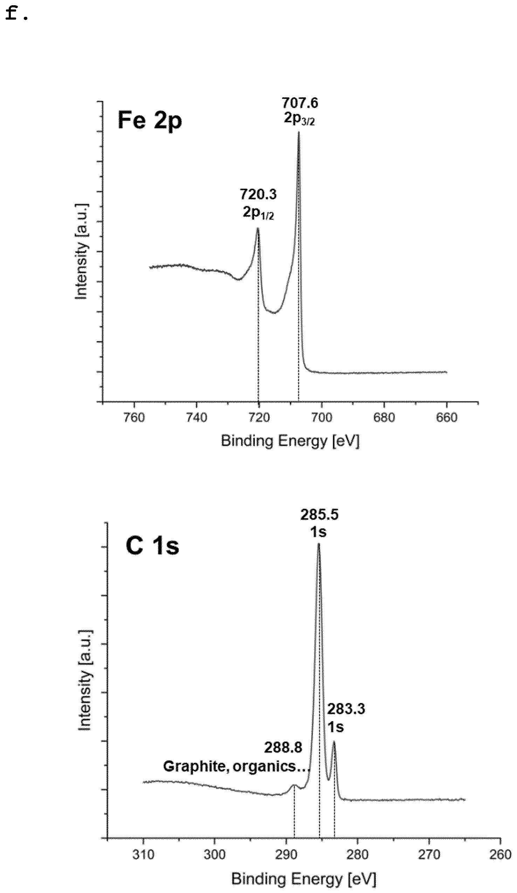

As expected, the incorporation of carbon into the Fe(0) lattice resulted in an increase of the NP size. The powder X-ray diffraction (PXRD) pattern of the obtained ICNPs showed the characteristic peaks of the pseudo-hexagonal Fe_{2.2}C carbide phase, and no sign of the presence of body-centered cubic (bcc) Fe(0) peaks (Figure 1c). The environment of Fe atoms was further characterized by using Mössbauer spectroscopy at 4 K (Figure 1d). The analysis revealed that the NPs contain a mixture of two different iron carbide phases Fe_{2.2}C (76 %) and Fe₅C₂ (20 %). Interestingly, the Fe₅C₂ phase was not visible in the XRD pattern, presumably due to its lack of crystallinity. Vibrating sample magnetometry (VSM) measurements on ICNPs at 300 K determined a magnetizations at saturation *Mₛ* of 122 A m² kg⁻¹ and a coercive field *H_{C}* of 80 mT, demonstrating the ferromagnetic behavior of these ICNPs at room temperature (Figure 1e). XPS measurements were further performed XPS characterization of Fe 2p and C 1s was performed similarly before and after catalysis in order to investigate the electronic structure of the ICNPs, verifying the presence of iron carbide phases. Both samples exhibit peaks at 707.6-707.4 eV (Fe 2p3/2) and 720.3-720.4 eV (Fe 2p1/2), confirming presence of iron carbides in both cases. Similarly, the samples exhibit peaks at 285.5 eV (sp² hybridized C), 288.8 eV for organic carbon species and 283.3 eV for Fe-C bonds, suggesting stability and electrical conductivity (Figure 1f). These findings are consistent with literature data.

### Example 2: Magnetocatalytic study

The catalytic performance of ICNPs activated by ACMFs was investigated for the hydrodeoxygenation of methylbenzoate (1) as a model substrate. The hydrodeoxygenation network of methylbenzoate is provided in Figure 3. Catalytic experiments were conducted under batch conditions using Ficher-Porter bottles as reactors (Table 1).

**Table 1. Energy efficiency estimation for two different heating techniques: Magnetic (70 mT, 350 kHz) and conventional heating method (200 °C).**

| | Magnetic (70 mT, 350 kHz) | Conventional (200 °C) |
|---|---|---|
| Time to target T°C (h) | 0 | 1.2 |
| Reactor T°C | 200 | 200 |
| Reaction time (h) | 4 | 4 |
| Energy input to reactor (Wh) | 239 | 2080 |
| Yield of **1a** (%) | >99 | 5 |
| Energy efficiency toward product formation (mmol MJ⁻¹) | 0.384 | 0.044 |

The exhaustive reduction of esters through magnetocatalysis (70 mT) absorb 239 Wh of power input and release it as thermal energy. The heat is generated almost instantaneously its working temperature (global T = 200 °C according to infra-red camera) and through standard condition of 70 mT, 350 kHz, 3 bar H₂, 4 h, substrate 1 was fully converted with products 1a in quantitative yield and high selectivity. This was calculated based on information that heat generation by ICNPs under ACMF considering 10 mg of ICNPs as catalyst amounts, which accounts for 7 mg of Fe. The SAR of ICNPs at 70 mT, 100 kHz yield to SAR = ca. 2430 W/g_{Fe}. For 70 mT and 350 kHz would thus result to ca. 8507 W/g_{Fe} (based on approximation of the Stoner-Wohlfrath model), meaning 59.55 W of heat released by ICNPs account for 239 Wh.

Whereas for the conventional heat method, using power meter, the energy consumption until the temperature was reached to 200 °C took 80 min that consumed 657 Wh. The reactor was maintained for 4 h more hours under 200 °C and consumed another 1423 Wh. Thus, for much lower catalytic performance, conventional heating consumed ca. 2080 Wh, while the magnetically heated catalyst consumed only 239 Wh and yielded full conversion of methyl benzoate to toluene (Table 1).

### Solvent effects

In typical experiments, methylbenzoate (**1**) was dissolved in a solvent (0.5 mL) and reacted in the presence of the catalyst (ICNPs) under H₂ (3 bar) and ACMF (*f* = 350 kHz, µ₀*H*ₘₐₓ = 70 mT). An infrared camera was used to systematically determine the temperature reached by the Fischer-Porter bottle as a result of the dissipation of thermal energy from the ICNPs catalyst under reaction conditions. This reactor temperature is later referred to as "global temperature". No stirring was implemented and mixing was insured only through convection.

The solvent plays a role in solution magnetocatalysis, as besides the solubilisation of substrates and products, solvent properties (e.g. boiling point, viscosity, etc.) can influence the interactions between substrate and active sites. Therefore, a series of solvent of different boiling points was tested under standard conditions (Figure 3a).

Interestingly, the global temperature was similar in all cases (ca. 200 °C), irrespective of the boiling point of the solvent. Monitoring the boiling of the solvents under these conditions, the surface temperature of the ICNPs could be estimated to ca. 330 °C, demonstrating that ICNPs can be seen as hot spots in a colder environment (see Table 2 for details).

**Table 2. Variation of amplitude field in screening of conditions and catalysts for the hydrodeoxygenation of 1.**

| Entry | Solvent/Solid | Boiling/ Melting Point [°C] | Tglobal [°C] | Boiling/ Melting upon magnetic induction? |
|---|---|---|---|---|
| 1 | Heptane | 98 | 201 | Yes |
| 2 | Mesitylene | 165 | 205 | Yes |
| 3 | Decalin | 186 | 198 | Yes |
| 4 | Dodecane | 216 | 197 | Yes |
| 5^{a} | Propylene carbonate | 242 | 202 | Yes |
| 6 | Tetradecane | 254 | 199 | Yes |
| 7 | Sulfolane | 285 | 204 | Yes |
| 8 | Hexadecane | 287 | 202 | Yes |
| 9 | Tetraethylene glycol | 327 | 201 | Yes |
| 10 | Mg(OH)₂ | 350 | 199 | No |

Local temperature at solvent/ICNPs interface estimated from local boiling/gas bubble formation of respective solvent. For entry 10, 50 mg of Mg(OH)₂ were used. Global temperatures were determined by IR camera. ^{a}=experiment was conducted for only 30 min as pressure increased to +2 bar due to decomposition of propylene carbonate to CO₂.

For solvents of relatively low boiling points (heptane and mesitylene), the conversion of **1** remained negligible presumably due to mass transfer limitation related to the Leidenfrost effect. For higher boiling solvents, good substrate conversions (49-64%) were observed after 1 h, giving selectively toluene (**1a**) as the only product. Exceptions to this trend are propylene carbonate and sulfolane which decomposed under these conditions, and tetraethylene glycol which high viscosity presumably severely hindered substrate transport to the active sites. Thus, decalin was selected as the cheaper option among suitable solvents for the rest of the study. Recording of a time profile under standard conditions showed the progressive conversion of **1** to **1a** over time (estimated initial rate r₀(**1**=>**1a**) = 0.44 min⁻¹), without detection of any intermediate. Quantitative yield of **1a** was reached after 4 h, and prolonging the reaction to 8 h did not change the product distribution, outlining the excellent selectivity of the ICNPs catalysts toward toluene without further hydrogenation of the arene ring.

### Mechanism

To gather insight into the mechanism, the potential intermediates benzaldehyde (**1b**) and benzylalcohol (**1c**) were used as substrates, leading to apparent initial hydrodeoxygenation rates of r₀ (**1b**=>**1a**) = 0.56 min⁻¹ and r₀(**1c**=>**1a**) = 1.66 min⁻¹, respectively (Figure 11). Again, **1c** was not observed as an intermediate in the hydrodeoxygenation of **1b.** These values indicate that the hydrodeoxygenation of **1b** and **1c** are substantially faster than that of 1, consistent with the absence of detected intermediate at any point of the reaction. The mechanism thus most likely proceeds through the relatively slow conversion of **1** to **1b,** followed by a faster conversion of **1b** to **1c,** and an even faster hydrodeoxygenation of **1c**. The proposed mechanism is consistent with the observation of methane, CO and CO₂ in the gas phase arising from the decomposition of methanol under these conditions (Figure 4b).

Interestingly, a time profile was recorded while regularly stopping and re-starting the ACMF, resulting in a perfectly concomitant stop and re-start of the catalytic activity (Figure 3c). This illustrates the extremely fast heating and cooling of the catalyst activated by magnetic induction, and the systems excellent adaptivity to fluctuations in electricity supply, a feature of strategic interest when considering the use of alternative renewable energy sources. In addition to that and among benefits of induction heating includes improved energy efficiency and lower energy cost (0.384 M/J) as compared to the conventional heating systems (0.044 M/J) (Table 1) via considering solely the global temperature of the solution as a reference point in temperature.

### Investigating ACMF amplitude

Decreasing the ACMF amplitude to 66, 56, and 50 mT led to milder heating of the ICNPs and slower reactions, as evidenced by incomplete conversions (75%, 45%, and 30% respectively) after 4 h of reaction (Table S3).

**Table 3. Variation of amplitude field in screening of conditions and catalysts for the hydrodeoxygenation of 1.**

| Entry | *µ*₀Hₘₐₓ | Global Temperature [°C] | Time of the reaction [h] | Selectivity [%] | Yield 1a [%] |
|---|---|---|---|---|---|
| 1 | 55.6 | 135 | 4 | >99 | 45 |
| 2 | 65.7 | 150 | 4 | >99 | 75 |
| 3 | 70 | 200 | 4 | >99 | >99 |
| 4 | 74 | 225 | 4 | >99 | >99 |

| | | | | | |
|---|---|---|---|---|---|
| Reaction conditions: methyl benzoate **1** (44.9 mg, 0.33 mmol), ICNPs (10 mg, 0.125 mmol of Fe), decalin (0.5 mL), H₂ (3 bar), magnetic field (350 kHz). The product selectivity is >99%. Product yields determined by GC-FID using tetradecane as the internal standard. Global temperature determined by IR camera | | | | | |

Increasing it to 74 mT provided quantitative yield of **1a,** without any detectable loss of selectivity. Using Fe(0)or FeₓO_{y} NPs of similar size as catalysts (Figure 5a) led to milder global temperatures (160 °C and 91 °C, respectively), reflecting the lower magnetic heating power (SAR) of these NPs. As a result, poor conversions were observed (53% and 0%, respectively).

The same NPs were tested under conventional heating to eliminate the dependence of the observed reactivity on NPs magnetic properties. Interestingly, none of these catalysts showed substantial activity at 200 °C (the global temperature observed in magnetocatalysis). Raising the temperature to 350 °C - the estimated surface temperature of ICNPs during magnetocatalysis - resulted in striking differences in the performance of the three catalyses, with ICNPs being found much more active (76% yield of **1a**) than metallic Fe NPs (21% yield of **1a**) and oxidized Fe NPs (4% yield of **1a**) (see Figure 5).

### Reusability and stability

Owing to their magnetic properties, ICNPs can be very easily separated from the product solution upon application of an external permanent magnet. Their reusability and stablity in magnetocatalysis and under conventional heating at 350 °C was investigated under adapted conditions insuring incomplete substrate conversion (Figure 6).

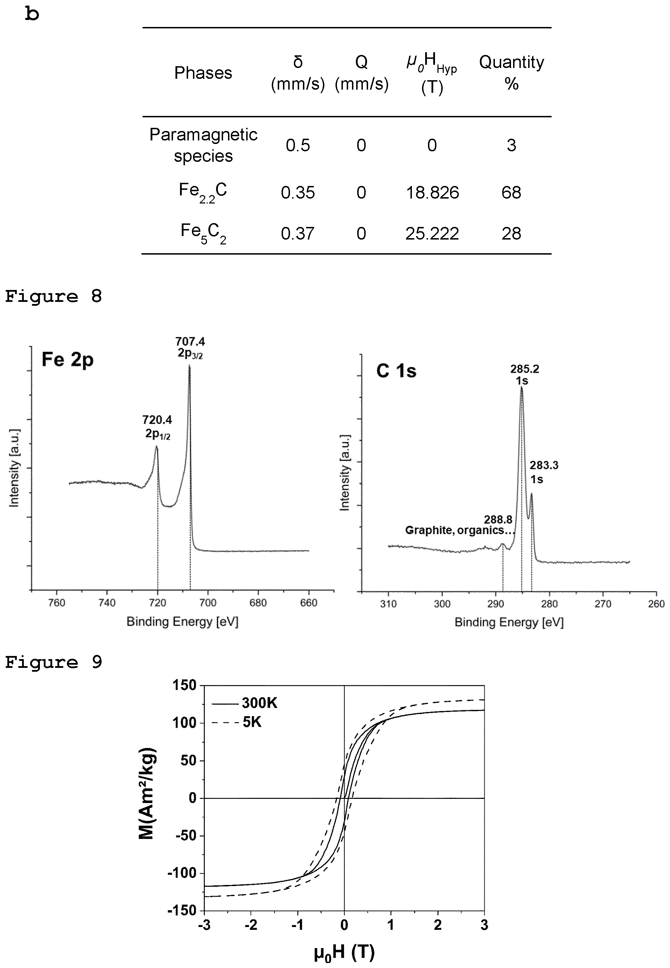

Satisfyingly, ICNPs could be recycled at least five times under magnetocatalytic conditions without any sign of deactivation (Figure 6a). Characterization of the ICNPs after 5 cycles by electron microscopy (Figure 6b), PXRD (Figure 6c), Mössbauer (Figure 7), XPS (Figure 8), and VSM (Figure 9) showed no noticeable change in NPs size, dispersion, structural, electronic and magnetic properties as compared to the pristine ICNPs (see detailed comparison in Table 4).

**Table 4. Summary of the systematic comparison of ICNPs before and after 5 cycle of methyl benzoate hydrodeoxygenation to toluene**

| | ICNPs prior recycle | | ICNPs after recycle | |
|---|---|---|---|---|
| NPs size | 12.4 ± 1.1 | | 12.3 ± 2.2 | |
| [nm] | | | | |
| Mossbauer Analysis: Content [%] | Paramagnetic species = 4% | | Paramagnetic species = 3% | |
| | Fe_{2.2}C=76% | | Fe_{2.2}C=68% | |
| | Fe₅C₂=20% | | | Fe₅C₂=28% |
| XPS analysis: Binding Energy [eV] | Fe 2 p | 2p_{1/2} = 720.3 | Fe 2 p | 2p_{1/2}=720.4 |
| | | 2p_{3/2} = 707.6 | | 2p_{3/2}=707.4 |
| | C 1S | Graphite, organics = 288.8 | C 1S | Graphite, organics = 288.8 |
| | | 1 s = 285.5 | | 1 s = 285.2 |
| | | 1 s = 283.3 | | 1 s = 283.3 |
| | Mₛ [Am²/kg] | Mₛ (300 K)= 122 | Mₛ [Am²/kg] | Mₛ (300 K)= 117 |
| | | Mₛ (5 K)= 132 | | |
| | | | | Mₛ (5 K) = 131 |
| | Mᵣ [Am²/kg] | Mᵣ (300 K)= 28 | Mᵣ [Am²/kg] | Mᵣ (300 K)= 30 |
| VSM Analysis | | Mᵣ (5 K) = 50 | | Mᵣ (5 K) = 46 |
| | Mᵣ/Mₛ [%] | Mᵣ/Mₛ (300 K) = 23.0 | Mᵣ/Mₛ [%] | Mᵣ/Mₛ (300 K) = 25.6 |
| | | Mᵣ/Mₛ (5 K) = 37.9 | | Mᵣ/Mₛ (5 K) = 35.1 |
| | H_{c} [mT] | H_{c} (300 K) = 80 | H_{c} [mT] | H_{c} (300 K) = 88 |
| | | H_{c} (5 K) = 180 | | H_{c} (5 K) = 166 |

ICP-OES showed negligible leaching of Fe in product solutions (1.5 - 3.9 µg/L, Table 5).

**Table 5. ICP-MS analysis of reaction solutions after each cycle of 2 h under standard conditions (recycle refers to Figure 6a).**

| **Reaction cycle** | **Fe concentration [µg/L]** |
|---|---|
| 1 | 1.49 ± 3.57 |
| 2 | 2.85 ± 3.65 |
| 3 | 3.05 ± 10.15 |
| 4 | 3.85 ± 5.20 |
| 5 | 3.27 ± 4.13 |

In striking contrast, catalytic performance rapidly declined upon use of ICNPs under conventional heating at 350 °C, with a loss of most of the toluene yield already after the first catalytic cycle (Figure 6d). STEM-HAADF showed aggregated and coalescenced ICNPs (Figure 6e), while PXRD revealed a complete transition from the initial Fe_{2.2}C crystallographic phase to crystalline Fe₅C₂. These results evidence an unexpected superior stability of ICNPs used under ACMFs as compared to conventional heating at a similar temperature. Without wishing to be bound by theory, the inventors consider it possible that the self-heating of ICNPs at high temperature (ca. 350 °C) in a colder liquid environment (ca. 200 °C) results in a hardening of the NPs, following a process comparable to induction hardening of steel in metallurgy.

### Testing a variety of substrates

The versality of the ICNP catalysts activated by magnetic induction was explored for a broad scope of aromatic esters (Table 6).

**Table 6. Magneto-catalytic hydrogenation of various esters using ICNPs.**

| | | | | | |
|---|---|---|---|---|---|
| 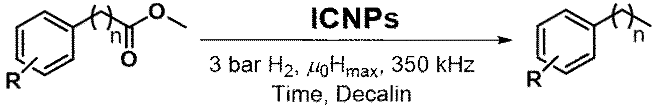 | | | | | |

| **Substrate** | ***µ₀*Hₘₐₓ** (mT) | **Time** (h) | **Conversion** (%) | **Product** | **Yield** (%) |
|---|---|---|---|---|---|
| 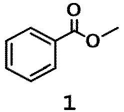 | 70 | 4 | >99 | 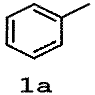 | >99 |
| 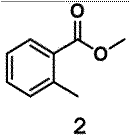 | 70 | 4 | >99 | 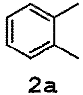 | 92 |
| 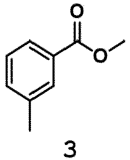 | 70 | 4 | >99 | 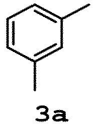 | 95 |
| 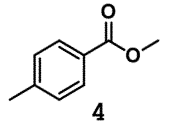 | 70 | 4 | >99 | 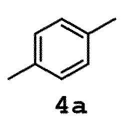 | 96 |
| 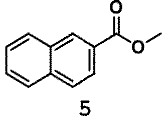 | 70 | 18 | >99 | 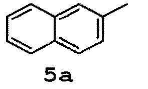 | 88 |
| 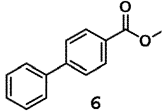 | 70 | 18 | >99 | 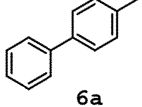 | 91 |
| 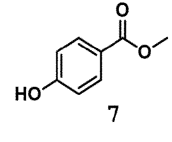 | 70 | 18 | >99 | 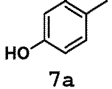 | 17 |
| | | | | 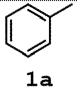 | 64 |
| 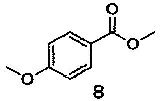 | 70 | 18 | >99 | 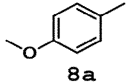 | >99 |
| 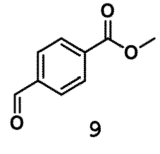 | 70 | 18 | >99 | 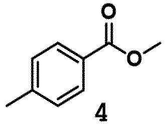 | 56 |
| | | | | 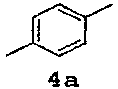 | 37 |
| 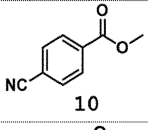 | 70 | 18 | >99 | 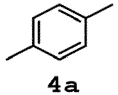 | 96 |
| 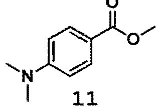 | 70 | 18 | >99 | 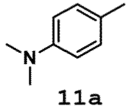 | 88 |
| 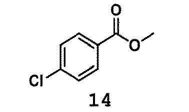 | 70 | 18 | 32 | 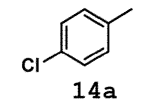 | 24 |
| 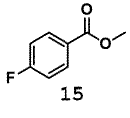 | 70 | 18 | >99 | 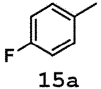 | 78 |
| | 65.7 | 18 | >99 | 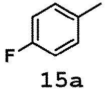 | 98 |
| 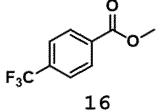 | 70 | 18 | >99 | 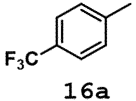 | 84 |
| 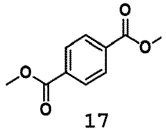 | 70 | 20 | >99 | 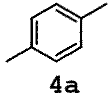 | 85 |
| 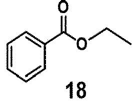 | 70 | 4 | >99 | 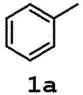 | 98 |
| 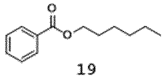 | 70 | 18 | >99 | 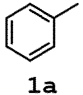 | >99 |
| | | | | 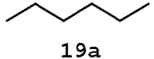 | 61 |
| 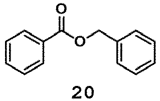 | 70 | 18 | >99 | 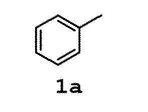 | 91 |
| 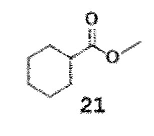 | 70 | 4 | >99 | 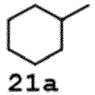 | 60 |
| | | | | 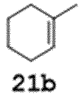 | 20 |
| | | | | 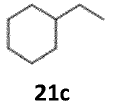 | 10 |
| 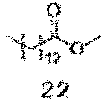 | 70 | 4 | >99 | 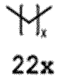 | 99 |

| | | | | | |
|---|---|---|---|---|---|
| Reaction conditions: substrate (0.33 mmol), ICNPs (10.0 mg, 0.125 mmol Fe), decalin (0.5 mL), H₂ (3 bar) under 4 h (unless stated as * = under 18 h). GC product yields determined by GC-FID using tetradecane as the internal standard. | | | | | |

The versality of ICNPs as magnetocatalysts for the selective hydrodeoxygenation of aromatic esters was explored through a broad substrate scope (Table 6). Satisfyingly, substrates **1-8** with electron donating substituents on the phenyl were all effectively hydrodeoxygenated, giving the corresponding desired toluene derivatives in excellent yields (88 - >99%). The position of the substituent on the ring did not affect catalytic performance under these conditions, as shown from *o-, m-,* and *p*-methyl (methylbenzoate) **(2-4).** Interestingly, the methoxy functionality was fully preserved in substrate **8.** In contrast, the hydroxy in **7** was fully cleaved, which is a promising observation for potential application of ICNPs to the selective hydrodeoxygenation of phenol derivatives. Most substrates bearing electron-withdrawing functionalities at the phenyl were also hydrodeoxygenated in high yields and selectivities. Hydrodefluorination could be mostly prevented in substrate **15** by lowering the ACMF amplitude. Interestingly, formyl, hydroxy, and nitrile substituents were removed under these conditions (cf. substrates **7, 9,** and **10,** respectively). Importantly, the PET model substrate dimethyl terephthalate **(17)** was converted to para-xylene **(4a,** 85%), substantiating the potential of this catalyst and approach for the conversion of waste polymers into valuable aromatic compounds. Finally, replacing the methyl side chain by bulkier substituents (e.g. ethyl **(18),** hexyl **(19),** and benzyl **(20))** still resulted in quantitative yields of the desired products.

In conclusion, ferromagnetic ICNPs are prepared, characterized, and applied to the challenging hydrodeoxygenation of aromatic esters. The intense thermal energy generated by ACMFs at the ICNPs enables the selective reduction of a wide variety of aromatic esters to toluene derivatives under low H₂ pressure (3 bar) and moderate reactor temperature (ca. 200 °C). Comparable performance could not be reached under conventional heating, even at 350 °C. Interestingly, ICNPs were found more active than Fe(0) and FexOy NPs for this transformation. ICNPs were found recyclable under magnetocatalytic conditions, with a robustness unprecedented for magnetic NPs used in liquid phase catalysis. Most strikingly, ICNPs suffered from severe restructuration and deactivation when used under conventional heating at the same temperature, suggesting that ACMF exposure impacts ICNPs in a yet unobserved and unforeseen manner, thereby conferring them exceptionally stable structural and catalytic properties. The results collectively demonstrate the promising potential of magnetocatalysis to enable facile ester hydrodeoxygenation using only Fe-based catalysis without other metals or specific ligands/additives. They also reveal a striking and unexpected positive effect of ACMFs on the stability of ICNPs, that may constitute a new crucial argument in favour of the practical implementation of magnetocatalysis in laboratories and industries.

## Claims

1. Method of hydrodeoxygenation of a substrate, wherein the method comprises:
bringing a substrate and hydrogen into contact with a catalyst; and
applying an alternating current magnetic field with a frequency of 30-600 kHz and a field amplitude of 10-150 mT;
wherein:
the substrate comprises at least one ester moiety; and
the catalyst comprises iron-containing particles, preferably wherein the iron-containing particles are nanoparticles.

2. Method of hydrodeoxygenation of a substrate, wherein the method comprises:
bringing a substrate, a solvent, and hydrogen into contact with a catalyst; and
applying an alternating current magnetic field with a frequency of 30-600 kHz and a field amplitude of 10-150 mT;
wherein:
the catalyst comprises iron-containing particles, preferably wherein the iron-containing particles are nanoparticles;
the substrate comprises at least one alcohol, aldehyde, ketone and/or ester group, preferably at least one alcohol, aldehyde and/or ester group, more preferably at least one ester group;
the solvent has a boiling point of 180-300 °C, preferably wherein the solvent is decalin, dodecane, tetradecane or hexadecane; and
the at least one alcohol, aldehyde, ketone and/or ester is reduced to the corresponding alkane.

3. Method of hydrodeoxygenation according to any one of claims 1-2, wherein the iron-containing particles comprise:
i) iron phosphide nanoparticles;
ii) iron sulphide nanoparticles;
iii) iron carbide nanoparticles; and/or
iv) Fe(0) nanoparticles;
preferably wherein the iron-containing particles comprise iron carbide nanoparticles and/or Fe(0) nanoparticles.

4. Method of hydrodeoxygenation according to any one of claims 1-3, wherein the catalyst does not contain any noble metals.

5. Method of hydrodeoxygenation according to any one of claims 1-4, wherein the iron-containing particles comprise iron carbide nanoparticles, and wherein the iron carbide nanoparticles comprise Fe_{2.2}C,
optionally wherein the iron carbide nanoparticles further comprise Fe₅C₂.

6. Method of hydrodeoxygenation according to any one of claims 1-5, wherein the iron-containing particles comprise iron carbide nanoparticles,
wherein the iron carbide nanoparticles consist of:
Fe_{2.2}C and Fe₅C₂, optionally wherein the ratio of Fe_{2.2}C to Fe₅C₂, is 40-99 : 1-60, preferably 50-99 : 1-50, more preferably 60-99 : 1-40; or
Fe_{2.2}C.

7. Method of hydrodeoxygenation according to any one of claims 1-6, wherein the catalyst consists of iron-containing nanoparticles,
preferably wherein:
i) the iron-containing nanoparticles comprise iron carbide nanoparticles and/or Fe(0) nanoparticles, preferably iron carbide nanoparticles;
ii) the iron-containing nanoparticles comprise:
50-100 wt%, preferably 80-100 wt%, of iron carbide nanoparticles and/or Fe(0) nanoparticles, preferably iron carbide nanoparticles, and
0-50 wt%, preferably 0-20 wt% of iron oxide nanoparticles.

8. Method according to any one of claims 1-7, wherein the catalyst consists of iron-containing nanoparticles, wherein the iron-containing nanoparticles consist of iron carbide nanoparticles and/or Fe(0) nanoparticles, preferably iron carbide nanoparticles.

9. Method according to any one of claims 1-8, wherein:
the field amplitude is 15-150 mT, preferably 50-100 mT, more preferably 65-90 mT, even more preferably 65-75 mT, most preferably 70 mT; and/or
the frequency is 150-450 kHz, preferably 250-400 kHz, more preferably 300-400 kHz, most preferably 350 kHz;
preferably wherein the field amplitude is 65-75 mT and the frequency is 300-400 kHz.

10. Method according to any one of claims 1-9, wherein the hydrogen is pressurised at 1-100 bar, preferably 1-20 bar, more preferably 1-10 bar, even more preferably 1-5 bar, most preferably 2-4 bar.

11. Method according to any one of claims 1 and 3-10, wherein the ester is reduced to the corresponding alkane.

12. Method according to any one of claims 1 and 3-11, wherein the hydrodeoxygenation is carried out in a solvent, preferably wherein:
i) the solvent has a boiling point of 80-350 °C, preferably 120-340 °C, more preferably 160-330 °C, even more preferably 170-320 °C, most preferably 180-300 °C; and/or
ii) the solvent has a viscosity of 0.1-5 mPa·s, preferably 0.5-3 mPa·s.

13. Method according to claim 12, wherein the solvent is a hydrocarbon-based solvent,
preferably wherein the hydrocarbon-based solvent is a linear or branched C₇-C₂₀ hydrocarbon-based solvent,
more preferably wherein the solvent is tert-butanol, dichloroethane, DMF, DMSO, dioxane, xylenes, an ionic liquid such as an imidazolium-based ionic liquid or a phosphonium-based liquid, supercritical CO₂, 2-methyl THF, decalin, dodecane, tetradecane, and hexadecane,
most preferably wherein the solvent is decalin, dodecane, tetradecane or hexadecane.

14. Use of a catalyst in a hydrodeoxygenation reaction, wherein the hydrodeoxygenation reaction involves:
bringing a substrate, a solvent, and hydrogen into contact with the catalyst; and
applying an alternating current magnetic field with a frequency of 30-600 kHz and a field amplitude of 10-150 mT, preferably a frequency of 300-400 kHz and a field amplitude of 65-75 mT;
wherein:
the catalyst comprises iron-containing nanoparticles, wherein the iron-containing nanoparticles comprise iron carbide nanoparticles and/or Fe(0) nanoparticles;
the substrate comprises at least one alcohol, aldehyde, ketone and/or ester group, preferably at least one alcohol, aldehyde and/or ester, preferably an ester;
the solvent has a boiling point of 180-300 °C, preferably wherein the solvent is decalin, dodecane, tetradecane or hexadecane;
the at least one alcohol, aldehyde, ketone and/or ester is reduced to the corresponding alkane.

15. Use of a catalyst in a hydrodeoxygenation reaction according to claim 14, wherein:
the catalyst consists of iron-containing nanoparticles; and/or
the catalyst does not contain any noble metals; and/or
the iron-containing nanoparticles comprise iron carbide nanoparticles comprising Fe_{2.2}C and optionally Fe₅C₂; and/or
the iron-containing nanoparticles comprise iron carbide nanoparticles and the iron carbide nanoparticles consist of Fe_{2.2}C and Fe₅C₂, optionally wherein the ratio of Fe_{2.2}C to Fe₅C₂, is 40-99 : 1-60, preferably 50-99 : 1-50, more preferably 60-99 : 1-40.
